Europäisches Patentamt

European Patent Office

Office européen des brevets

(11) Publication number: **0 200 555**
**A2**

# EUROPEAN PATENT APPLICATION

(21) Application number: 86303339.5

(22) Date of filing: 01.05.86

(51) Int. Cl.⁴: **A 63 B 23/00**
**A 63 B 21/30**

(30) Priority: 01.05.85 GB 8511056

(43) Date of publication of application:
05.11.86 Bulletin 86/45

(84) Designated Contracting States:
CH DE FR IT LI SE

(71) Applicant: Bell, John
71 Honeywell Grove
Barnsley South Yorkshire S71 1QH(GB)

(72) Inventor: Bell, John
71 Honeywell Grove
Barnsley South Yorkshire S71 1QH(GB)

(74) Representative: Harrison, Michael Robert et al,
Urquhart-Dykes & Lord 5th Floor Tower House Merrion
Way
Leeds, LS2 8PA(GB)

(54) Training aid.

(57) A training aid for use by handicapped people comprises
a flexible resilient pad 1 connected by two wires 2, 3 to a plug
4. An indicator unit 15 for use with the pad 1 incorporates
two circuits, a first circuit which activates a bleeper and/or
LED when the pad 1 is either pressed or released, and a
second circuit which activates an array of LEDS L2 to L11 to
give a visual indication of the degree of pressure applied to
the pad 1.

Fig.1.

EP 0 200 555 A2

Croydon Printing Company Ltd.

# TRAINING AID

The present invention relates to an aid for use in training a handicapped person to apply pressure with his hand or finger to a particular location.

Often handicapped people have great difficulty in lifting objects since they cannot exert a strong enough gripping force on the object, and they also experience difficulty in locating the object sufficiently accurately.

It is therefore the purpose of the present invention to provide a training aid which is able to detect when pressure is applied to it, and to indicate this to the person. Such a device would be useful in certain types of physiotherapy, especially paediatric physiotherapy.

According to the present invention there is provided a training aid comprising a flexible resilient pad having first and second electrical connections thereto, and means for producing a signal between said first and second electrical connections which is indicative of the pressure applied to the pad.

The aid may be used in the form of a switch which is open when no pressure is applied to the pad, but which may be closed by the application of pressure to the pad. In this case, the pad preferably comprises a first contact member for connecting to the first electrical connection, a second contact member for connecting to the second electrical connection, said first and second contact members being contained within a flexible insulating housing in such a way that the contacts are in substantially parallel alignment, one vertically above the other, the contact members being separated by at least one layer of resilient material around the periphery of at least one of the contact members, such that the contacts are movable between a first position adopted when no pressure is being applied to the pad and in which there is

r o electrical connection between the two contact members, and a second position resulting from pressure being applied to the pad anywhere within the area defined by the periphery of the contact members and in which position electrical connection is made between the two contact members.

Preferably the first and second contact members are both made of metal foil.

Preferably the metal foil of the contact members is thin aluminium foil.

Preferably the aluminium foil is conditioned to improve the strength thereof.

Preferably the aluminium foil is conditioned by wrinkling the foil to introduce a number of creases.

Preferably the wrinkling is carried out in each of two directions, these being substantially at right angles.

Thus, in this way, the lifetime of the pad is considerably longer than if the foil were not so conditioned, the wrinkling serving to increase the resistance of the foil to tearing as the pad is pressed.

Preferably the resilient material around the periphery of the contact members is made of foam.

Preferably the thickness of the foam may be varied to impart pressure sensitivity to the pad.

Such a switch can be used alone, or can be combined with a number of similar switches and can be coupled to a computer to replace a conventional keyboard.

Alternatively, the aid may be used as a pressure sensitive pad, in which case it preferably comprises a first contact member for connecting to the first electrical connection, a second contact member for connecting to the second electrical connection, said first and second contact members being contained within a flexible insulating housing in such a way that the contacts are in substantially parallel alignment one vertically above the other, and located between said first and second contact members a third member of resilient flexible material which, when compressed, results in a change in voltage between said first and second electrical connections.

Preferably the first and second contact members are made of copper foil.

Preferably the thickness of the copper foil is approximately forty thousandths of an inch.

Preferably the third member is made of carbon impregnated felt.

In either case, the flexible insulating housing is preferably made of plastics material. Preferably the housing includes at least one transparent pocket to allow the pad to be temporarily marked.

Preferably the first and second electrical connections to the contact members are made to the underside of the contact members, between the contact members and the housing.

Preferably, the aid includes an indicator unit for connecting to the pad, said unit including a first electronic circuit for use with a pad acting as a switch, said circuit having two modes of operation; a normal mode in which an audio or visual indicator or both operates when the pad is pressed, and the reverse mode in which the audio or visual indicator or both operates until the switch is pressed, when the or each indicator is rendered inoperative.

A variable delay may also be incorporated in the circuit whereby the indicator operates after a certain time period has expired, unless the switch is pressed.

Preferably the unit has two input terminals, one for the normal mode and one for the reverse mode.

Preferably the unit has high input resistance.

Preferably the unit runs from a nine volt supply.

Preferably, the indicator unit also includes a second electronic circuit for use with the pressure sensitive pad which measures the voltage across the first and second electrical connections and gives a visual or audio indication of the value of said voltage.

Embodiments of the present invention will now be described by way of example only with reference to the accompanying drawings in which:

Figure 1 is a plan view of a pad according to the present invention;

Figure 2 is a sectional view of a pad forming a first embodiment of the present invention;

Figure 2a is an equivalent circuit to the pad shown in Figure 2;

Figure 3 is a sectional view of a pad forming a second embodiment of the present invention;

Figure 3a is an equivalent circuit of the pad shown in Figure 3;

Figure 4 is a perspective view of an indicator unit forming part of the present invention; and,

Figure 5 is a circuit diagram of the indicator unit shown in Figure 4.

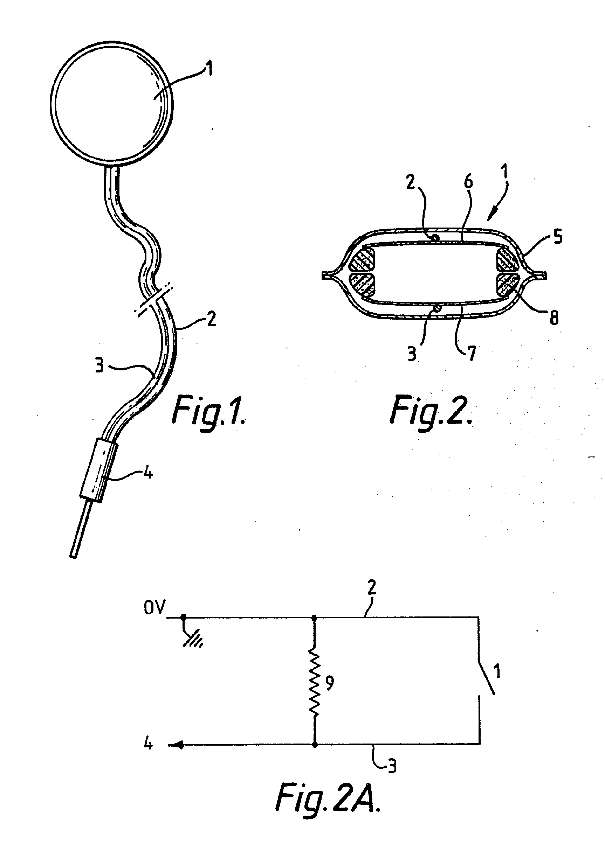

Referring to Figure 1, an aid according to the present invention includes a flexible resilient pad 1 connected by two wires 2, 3 to a plug 4 which can be plugged into an indicator unit to be later described in more detail.

In one embodiment of the invention, the construction of the pad 1 is as shown in Figure 2. A flexible insulating housing 5 surrounds two pieces of aluminium foil 6, 7 separated by pieces 8 of foam or other resilient material around the periphery of the pad. Wires 2, 3 are connected to the foil pieces 6, 7 to form a circuit as shown in Figure 2a.

When no pressure is applied to the pad, switch 1 is open and the indicator unit is measuring the voltage across the resistor 9 which is wired in parallel between the two

020055

wires 2, 3.

However, when the switch 1 is closed, plug 4 is earthed and this is displayed on the indicator unit by a visual or audio signal.

The aluminium foil 6, 7 is conditioned by wrinkling in each of two directions at right angles to increase its strength and thus the lifetime of the pad.

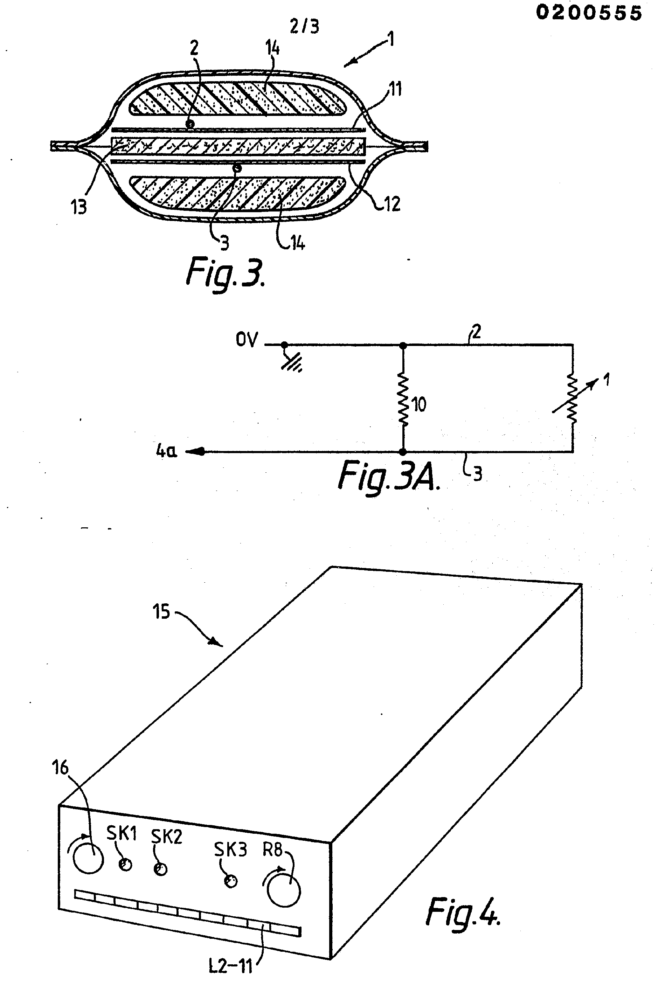

In another embodiment, the construction of the pad is as shown in Figure 3. Two pieces of copper foil 11, 12 have wires 2, 3 attached, and are separated by a piece of carbon impregnated felt 13 to form the circuit shown in Figure 3a. The carbon impregnated felt effectively acts as a variable resistor, its resistance dropping as it is squeezed. The varying voltage on plug 4a is measured by the indicator unit which produces a visual or audio signal indicative of the pressure applied to the pad 1. Foam padding 14 may also be included to increase the resilience of the pad.

Referring now to Figure 4, an indicator unit 15 for use with both types of pads described above comprises two sockets SK1 and SK2 which each accept plug 4 of the pad shown in Figure 2, and a third socket SK3 which accepts the plug 4a of the pad shown in Figure 3.

The unit also includes a delay control 16 and a sensitivity control R8. A multi-LED display L2 to L11 is used in conjunction with socket SK3 to give a visual indication of the amount of pressure applied to the pad shown in Figure 3.

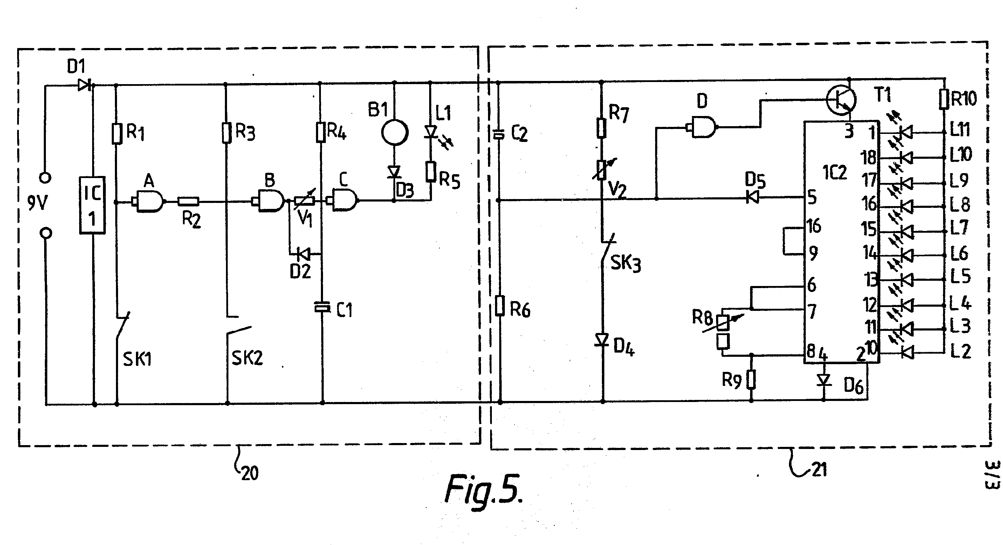

The circuit of the indicator unit 15 is shown in Figure 5 and comprises two parts 20, 21. As shown, the plug 4 of the pad shown in Figure 2 is plugged into the socket SK1 for the normal mode of operation in which a bleeper B1 and/or LED L1 is activated when the pad is pressed.

Two logic gates A, B (these can be either NAND or NOR gates) are connected to the sockets SK1 and SK2 respectively, and to change the mode of operation to reverse mode in which LED L1 and/or bleeper B1 are activated until the pad is pressed, the plug 4 is simply removed from socket SK1 and plugged into socket SK2, thus effectively by-passing gate A.

In the normal mode of operation, when the logic state of the output of a third logic gate C is high, the LED L1 and bleeper B1 are inoperative. When the pad is pressed, this logic state changes to low and current flows through bleeper B1 and LED L1 until the output of C changes back to high again. The operation is similar for the reverse mode of operation except that the output of gate C is changed from low to high by pressing the pad (this is represented by variable voltage V1).

The second part 21 of the circuit is concerned with the pad shown in Figure 3 and incorporates a bar graph display driver IC2 which is driven by a transistor T1.

In operation, plug 4a of the pad shown in Figure 3 is plugged into socket SK3. When the pad, which is represented by variable voltage V2 is pressed, the voltage at pin 5 of IC2 is varied which causes different LEDs of the array L2 to L11 to light up, according to the degree of pressure applied to the pad.

Resistor R8 can be varied (see sensitivity control R8 in Figure 4) to alter the sensitivity of this device to suit the particular application.

Either part 20, 21 of the circuit may be used independently, or they may be used together as required.

Pads as shown in Figure 2 may also be connected together in an array and coupled to a computer to provide an alternative to a computer keyboard.

0200555

CLAIMS

1. A training aid characterised in that it comprises a flexible resilient pad (1) having first and second electrical connections (2,3) thereto, and means (20,21) for producing a signal between said first and second electrical connections which is indicative of the pressure applied to the pad.

2. A training aid according to Claim 1 characterised in that the pad comprises a first contact member (6) for connecting to the first electrical connection (2), a second contact member (7) for connecting to the second electrical connection (3), said first and second contact members being contained within a flexible insulating housing (5) in such a way that the contacts are in substantially parallel alignment, one vertically above the other, the contact members being separated by at least one layer of resilient material (8) around the periphery of at least one of the contact members, such that the contacts (6,7) are movable between a first position adopted when no pressure is being applied to the pad (1) and in which there is no electrical connection between the two contact members, and a second position resulting from pressure being applied to the pad (1) anywhere within the area defined by the periphery of the contact members, and in which position electrical connection is made between the two contact members (6,7).

3. A training aid according to Claim 2 characterised in that the first and second contact members (6,7) are made of aluminium foil.

4. A training aid according to Claim 3 characterised in that the foil is conditioned to improve the strength thereof by wrinkling the foil in each of two directions to substantiate right angles to each other.

5. A training aid according to any of Claims 2 to 4 characterised in that the resilient material (8) around the periphery of the contact members is foam material.

6. A training aid according to Claim 1 characterised in that the pad comprises a first contact member (11) for connecting to the first electrical connection (2), a second contact member (12) for connecting to the second electrical connection (3), said first and second contact members being contained within a flexible insulating housing (5) in such a way that the contacts are in substantially parallel alignment one vertically above the other, and located between said first and second contact members a third member (13) of resilient flexible material which, when compressed, results in a change in voltage between said first and second electrical connections.

7. A training aid according to Claim 6 characterised in that the first and second contact members (11,12) are made of copper foil.

8. A training aid according to Claim 6 or Claim 7 characterised in that the third member (13) is carbon impregnated felt.

9. A training aid according to any of the preceding Claims characterised in that there is included an indicator unit (15) having a first electronic circuit (20) for use with a pad acting as a switch, the circuit having two modes of operation; a normal mode in which an audio or visual indicator or both operates when the switch is pressed, and a reverse mode in which the audio or visual indicator or both operates until the switch is pressed, when the or each indicator is rendered inoperative.

10. A training aid according to Claim 9 characterised in that the indicator unit (15) includes a second electronic circuit (21) for use with the pressure sensitive pad, the second electronic circuit measuring the voltage across the electrical input and output and giving a visual or audio indication of the value of said voltage.

Fig.1.

Fig.2.

Fig.2A.

**Fig.3.**

**Fig.3A.**

**Fig.4.**

*Fig.5.*